(19) Europäisches Patentamt
European Patent Office
Office européen des brevets

(11) EP 2 238 912 B1

(12) EUROPEAN PATENT SPECIFICATION

(45) Date of publication and mention of the grant of the patent:
**27.01.2016 Bulletin 2016/04**

(21) Application number: **10157867.2**

(22) Date of filing: **26.03.2010**

(51) Int Cl.:
***A61B 8/08*** (2006.01) ***G01S 7/52*** (2006.01)
***G01S 15/89*** (2006.01) *A61B 8/06* (2006.01)

# (54) Ultrasound system and method of providing color M mode image and brightness M mode image

Ultraschallsystem und Verfahren zur Bereitstellung eines Farb-M-Modus-Bildes und eines Hell-M-Modus-Bildes

Système à ultrasons et procédé pour la fourniture d'une image en mode M couleur et d'une image en mode M luminosité

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **07.04.2009 KR 20090029678**

(43) Date of publication of application:
**13.10.2010 Bulletin 2010/41**

(73) Proprietor: **Samsung Medison Co., Ltd.**
**Gangwon-do 25108 (KR)**

(72) Inventors:
- **Kim, Jong Sik**
**1003 Daechi-dong, Gangnam-gu**
**Seoul 135-851 (KR)**
- **Lee, Kwang Ju**
**1003 Daechi-dong, Gangnam-gu**
**Seoul 135-851 (KR)**

(74) Representative: **Schmid, Wolfgang**
**Lorenz & Kollegen**
**Patentanwälte Partnerschaftsgesellschaft mbB**
**Alte Ulmer Strasse 2**
**89522 Heidenheim (DE)**

(56) References cited:
**EP-A1- 1 498 746**
**US-A- 4 817 617**
**US-A1- 2003 045 795**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 238 912 B1

**Description**

## TECHNICAL FIELD

The present disclosure relates to ultrasound systems, and more particularly to an ultrasound system and a method of providing a color M mode image and a brightness M mode image corresponding to an M line set on a color Doppler mode image and a B mode (brightness mode) image.

## BACKGROUND

An ultrasound system has been extensively used for acquiring internal information of a target object due to its non-invasive and non-destructive nature. Since the ultrasound system may provide a high resolution image in real-time without any surgical treatment, it has proven to be very helpful in the medical profession.

Generally, the ultrasound system provides B mode (brightness mode) that shows a reflection coefficient of a ultrasound signal reflected from a target object in a 2-dimensional image, doppler mode showing an image of a moving target object using doppler effect, motion mode (M mode) showing a change of biometric information (e.g., brightness information) over time of a target object in a particular part of a B mode image, and elastic mode showing a difference of reaction between a target object with and without compression in an image.

Also, the ultrasound system sets an M line on the B mode image and the color doppler mode image, and provides the color M mode image and the brightness M mode image corresponding to the M line. The color M mode image may show how the blood flow changes over time on the M line. Typically, the M mode acquires ultrasound data within a particular time interval (i.e., sweep period) and displays an M mode image formed from the acquired ultrasound data. The sweep speed of the color M mode is commonly used in a range of 60 to 360Hz, which is not sufficient from the point of the time resolution. In the brightness M mode, assuming that the velocity of sound is 1540m/s and the depth is 15cm, the time required to acquire the ultrasound data corresponding to one scan line is very short, i.e., approximately 200$\mu$s, when the pulse repetition frequency (PRF) of the ultrasound is 5 KHz . Thus, the problem of the time resolution does not occur. In other words, the brightness M mode has no problem in acquiring the ultrasound data corresponding to one scan line. The color M mode acquires a plurality of ultrasound data on one scan line by repeatedly transmitting and receiving an ultrasound signal as many times as an average number of the data acquisition (an ensemble number) in the same direction. An ultrasound data acquisition period for acquiring ultrasound data of color M mode is inversely proportional to the sweep speed. For example, if the ultrasound data acquisition frequency (the PRF for radiating the ultrasound) is 2kHz and the average number of the data acquisition is 12, then the time for forming the color M mode image according to one scan line is 6000$\mu$s = 500$\mu$s (the ultrasound data acquisition frequency (2kHz)) x 12 (the average number). In such a case, the sweep speed should be less than 160Hz (1/6000$\mu$s). With this low speed, it is difficult to provide continuous color M mode. Also, as the average number is reduced to increase the sweep speed, the ultrasound data for forming the color M mode image should be reduced. This also degrades the quality of the color M mode image.

The EP 1 498 746 A1 discloses that by changing the quality of a display image, according to (a) the generation amount of the acoustic line data per unit time or (b) the number of display frames of an image, a resource control including the selection of the optimum drawing process algorithm is realized. Specifically, the two-dimensional drawing module (i) periodically reads acoustic line data, (ii) checks a scanning method of a physical structure of a probe and acoustic line data, and (iii) selects the optimum coordinate conversion algorithm. After that, the two-dimensional drawing module (i) selects an interpolation algorithm, based on a display rate, (ii) generates display data by performing a two-dimensional DSC process using the selected coordinate conversion algorithm and the like, (iii) performs a persistence process for the display data, a frame interpolating process for a "B mode" image data and a display color conversion process, and (iv) transmits the display data after such processes as described above to the display unit.

In the US 2003/0045795 A1 a method is provided to simultaneously acquire two ultrasound images. A first set of ultrasound pulses are transmitted at a first frame rate utilizing a first mode of operation. The echoes from the first set of ultrasound pulses are received. A second set of ultrasound pulses are transmitted at a second frame rate utilizing a second mode of operation. The first and second frame rates are different. The first set of ultrasound pulses defines an entire image, while the second set of ultrasound pulses defines a partial image. The echoes from the second set of ultrasound pulses are received, and the echoes from the first and second sets of ultrasound pulses are displayed as a single image.

In the US 4 817 617 a diagnostic imaging apparatus is disclosed in which an ultrasonic wave transmit/receive apparatus is used in common for both 2-dimensional B-mode imaging and for measurement of pulsed Doppler signals, by time sharing operation. The good real time characteristics of 2-dimensional B-mode imaging are retained, together with a sufficiently high data rate for pulsed Doppler measurement, through use of simple means for performing interpolation in the Doppler signals Ultrasonic wave transmit/receive means repetitively execute combinations of ultrasonic wave transmit/receive sequences, each combination consisting of a plurality of pulsed Doppler mode ultrasonic wave transmit/receive sequences and a single 2-dimensional B-mode imaging ultrasonic wave transmit/receive sequence. Demodulator means demodulate the Doppler signals, based on the received signals that are acquired during the pulsed Doppler mode ultrasonic wave transmit/receive sequences. Sampling means perform range gate sampling of the demodulated Doppler signals, and interpolation means interpolate sampling values into the Doppler signal to replace sampling values which are lost during execution of the 2-dimensional B-mode imagaing ultrasonic wave transmit/receive sequences. Doppler signal processing means perform processing of the sampling signals following interpolation. 2-dimensional B-mode imaging means process the received signal that is acquired by the 2-dimensional B-mode imaging sequences.

## SUMMARY

Embodiments of an ultrasound system and a method adapted to provide a color M mode image and a brightness M mode image with high quality based on an average number (an ensemble number) and a sweep period for acquiring the color M mode image are disclosed herein, the ultrasound system and the method being specified in claims 1 and 8, respectively.

## BRIEF DESCRIPTION OF THE DRAWINGS

| | |
|---|---|
| FIG. 1 | is a block diagram showing an illustrative embodiment of an ultrasound system. |
| FIG. 2 | is a block diagram showing an illustrative embodiment of an ultrasound data acquisition unit. |
| FIG. 3 | is a block diagram showing an illustrative embodiment of an image processor. |
| FIG. 4 | shows an example of an M line set on a B mode image and color doppler mode image. |
| FIGS. 5 and FIG. 6 | show an example of acquiring first pieces of ultrasound data and second pieces of ultrasound data. |
| FIGS.7 and 8 | show an example of the first pieces of ultrasound data and the second pieces of ultrasound data stored in a storage unit. |
| FIG. 9 | shows an example of third pieces of ultrasound data obtained through interpolation of the first pieces of ultrasound data. |

## DETAILED DESCRIPTION

A detailed description may be provided with reference to the accompanying drawings. FIG. 1 is a block diagram showing an illustrative embodiment of an ultrasound system 100. The ultrasound system 100 comprises a user input unit 110, an M line setting unit 120, a control unit 130, an ultrasound data acquisition unit 140, a storage unit 150, an image processor 160 and a display unit 170.

The user input unit 110 is implemented as a control panel, a mouse, a keyboard, etc., to allow a user to input M line setting information. The M line setting information comprises location information of the M line 440 set on a B mode (brightness mode) image 410 and a color doppler mode image 420, as illustrated in FIG. 4. The reference number 430 represents an area of interest for acquiring the color doppler mode image 420 in FIG. 4.

The M line setting unit 120 is configured to set the M line 440 on the B mode (brightness mode) image 410 and the color doppler mode image 420 according to the M line setting information provided by the user input unit 110, as illustrated in FIG. 4. Although in the aforementioned embodiment, the M line setting unit 120 has been explained as setting the M line on the B mode image and the color doppler mode image according to the M line setting information provided by the user input unit 110, the present invention is certainly not limited thereto. In another embodiment, the M line setting unit 120 may be configured to set the M line on the B mode image and the color doppler mode image according to a predetermined M line setting information. The control unit 130 is operable to control acquisition of an ultrasound data according to a sweep speed, a sweep period, an average number, an ultrasound data acquisition period and an ultrasound data acquisition time. The sweep period is a time period of displaying an ultrasound image and the sweep speed is the end point of every sweep period. The ultrasound data acquisition period is a time period of providing a first piece of ultrasound data. The ultrasound data acquisition period is equal to the sweep period divided by the average number. The first piece of ultrasound data includes ultrasound data for acquiring a color motion mode (CM mode) image corresponding to the M line. The ultrasound data acquisition time includes a time period expected for providing the first piece of ultrasound data (hereinafter referred to as first time) and a time period expected for providing a second piece of ultrasound data (hereinafter referred to as second time). The second piece of ultrasound data includes ultrasound data for acquiring a brightness M mode (BM mode) image corresponding to the M line. The control unit 130 calculates an aggregate time ($T_1 + T_2$) by adding the first time ($T_1$) to the second time ($T_2$), as illustrated in FIG 5 and FIG. 6. The control unit 130 compares the aggregate time ($T_1 + T_2$) with the ultrasound data acquisition period ($T_{CM}$). If the aggregate

time ($T_1 + T_2$) is less than the ultrasound data acquisition period ($T_{CM}$), then the control unit 130 controls the first piece of ultrasound data (CM) and the second piece of ultrasound data (BM) to be provided alternately, as illustrated in FIG. 5. If the aggregate time ($T_1 + T_2$) is equal to or greater than the ultrasound data acquisition period ($T_{CM}$), then the control unit 130 may control the first pieces of ultrasound data (CM) to be provided by the average number and then the second piece of ultrasound data (BM) to be provided once at the sweep speed (SP), as illustrated in FIG. 6.

Further, the control unit 130 may operate to control the setting of the M line and storage of the ultrasound data. Additionally, the control unit 130 operates to control formation and display of the CM mode image and the BM mode image according to the ultrasound acquisition period and the ultrasound acquisition time.

The ultrasound acquisition unit 140 is operable to transmit an ultrasound signal to a target object, receive the ultrasound signal reflected from the target object (i.e., ultrasound echo signal), and provide the ultrasound data according to the control unit 130.

FIG. 2 is a block diagram showing an illustrative embodiment of the ultrasound data acquisition unit 140. The ultrasound data acquisition unit 140 includes a transmit signal forming unit 210, a ultrasound probe 220 including a plurality of transducer elements, a beam former 230 and a ultrasound data forming unit 240.

The transmit signal forming unit 210 is operable to form a transmit signal to be applied to each of the transducer elements of the ultrasound probe 220. By way of a non-limiting example, the positions and focusing points of the transducer elements are considered in forming the transmit signal. In one embodiment, the transmit signal includes a first transmit signal for acquiring the CM mode image and a second transmit signal for acquiring the BM mode image.

The ultrasound probe 220 operates to convert the transmit signal provided by the transmit signal forming unit 210 into an ultrasound signal and transmit it to the target object. The ultrasound probe 220 further operates to receive the ultrasound echo signal reflected from the target object and form a receive signal. In one embodiment, when the first transmit signal is provided by the transmit signal forming unit 210, the ultrasound probe 220 operates to convert the first transmit signal into an ultrasound signal, transmit it to the target object, receive the ultrasound echo signal reflected from the target object and form a first receive signal. In one embodiment, when the second transmit signal is provided by the transmit signal forming unit 210, the ultrasound probe 220 operates to convert the second transmit signal into an ultrasound signal, transmit it to the target object, receive the ultrasound echo signal reflected from the target object and form a second receive signal.

In one embodiment, the beam former 230 is configured to form a first digital signal through analog-to-digital conversion of the first receive signal when the first receive signal is provided by the ultrasound probe 220. The beam former 230 performs receive-focusing upon the first digital signal in consideration of the positions and focusing points of the transducer elements of the ultrasound probe 220, and forms a first receive-focused signal thereby. In one embodiment, the beam former 230 forms a second digital signal through analog-to-digital conversion of the second receive signal when the second receive signal is provided by the ultrasound probe 220. The beam former 230 performs receive-focusing upon the second digital signal in consideration of the positions and focusing points of the transducer elements of the ultrasound probe 220, and forms a second receive-focused signal thereby.

In one embodiment, the ultrasound data forming unit 240 is configured to form the first piece of ultrasound data using the first receive-focused signal when the first receive-focused signal is provided by the beam former 230. In one embodiment, the ultrasound data forming unit 240 is configured to form the second piece of ultrasound data using the second receive-focused signal when the second receive-focused signal is provided by the beam former 230. Further, the ultrasound data forming unit 240 performs various signal processing (e.g., gain adjustment, filtering) to the first and second receive-focused signal for forming the ultrasound data.

Referring back to FIG. 1, the storage unit 150 is configured to store the first and second pieces of ultrasound data provided by the ultrasound data acquisition unit 140 according to the control unit 130. In one embodiment, the storage unit 150 is configured to store the first pieces of ultrasound data (CM) and the second pieces of ultrasound data (BM) in the order of being provided from the ultrasound data acquisition unit 140 according to the control unit 130, as illustrated in FIG. 7. In another embodiment, the storage unit 150 is configured to store the first pieces of ultrasound data (CM) and the second pieces of ultrasound data (BM) in the order of being provided from the ultrasound data acquisition unit 140 according to the control unit 130, as illustrated in FIG. 8.

Although in the aforementioned embodiment, the storage unit 150 has been explained as storing the first pieces of ultrasound data and the second pieces of ultrasound data, the present invention is certainly not limited thereto. In another embodiment, the storage unit 150 includes a first storage unit (not shown) for storing the first pieces of ultrasound data and a second storage unit (not shown) for storing the second pieces of ultrasound data.

The image processor 160 is operable to extract the first pieces of ultrasound data and the second pieces of ultrasound data in consideration of the sweep speed, the average number, the ultrasound data acquisition period and the ultrasound data acquisition time according to the control unit 130. The image processor 160 further operates to form the CM mode image and the BM mode image based on the extracted first and second pieces of ultrasound data.

FIG. 3 is a block diagram showing an illustrative embodiment of the image processor 160. The image processor 160 includes a determination unit 310, an extraction unit 320, a CM mode image forming unit 330 and a BM mode image forming unit 340.

The determination unit 310 is configured to form a determination result using the sweep speed, the average number, the ultrasound data acquisition period and the ultrasound data acquisition time. The determination unit 310 is configured to form first determination information if the ultrasound data acquisition time, which is the aggregate time ($T_1 + T_2$) calculated by adding the first time to the second time, is less than the ultrasound data acquisition period ($T_{CM}$), as illustrated in FIG. 5. In one embodiment, the determination unit 310 is configured to form second determination information if the aggregate time ($T_1 + T_2$) calculated by adding the first time to the second time is equal to or more than the ultrasound data acquisition period ($T_{CM}$), as illustrated in FIG. 6.

The extraction unit 320 is configured to extract the first pieces of ultrasound data and the second pieces of ultrasound data based on the sweep speed when the first determination information is provided by the determination unit 310. In one embodiment, the extraction unit 320 is configured to extract a predetermined number (e.g., 12) of the first pieces of ultrasound data ($CM_{18}$-$CM_7$) starting at the first piece of ultrasound data ($CM_{18}$), which is lastly stored during the sweep period, from the storage unit 150, as illustrated in FIG. 7. The extraction unit 320 further operates to extract the second piece of ultrasound data ($BM_{18}$), which is lastly stored during the sweep period, from the storage unit 150. In another embodiment, the extraction unit 320 is configured to extract a predetermined number (e.g., 12) of the first pieces of ultrasound data ($CM_{18}$-$CM_7$) starting at the first piece of ultrasound data ($CM_{18}$), which is lastly stored during the sweep period, from the storage unit 150, as illustrated in FIG. 7. The extraction unit 320 further operates to extract a predetermined number (e.g., 12) of the second pieces of ultrasound data ($BM_{18}$-$BM_7$) starting at the second piece of ultrasound data ($BM_{18}$), which is lastly stored during the sweep period, from the storage unit 150, as illustrated in FIG. 7.

The extraction unit 320 is configured to extract a predetermined number of the first pieces of ultrasound data and the second pieces of ultrasound data accumulated during the sweep period from the storage unit 150 when the second determination information is provided by the determination unit 310. In one embodiment, the extraction unit 320 is configured to extract a predetermined number (e.g., 12) of the ultrasound data (i.e., ten of the first pieces of ultrasound data ($CM_{16}$-$CM_7$) and two of the second pieces of ultrasound data ($BM_2$, $BM_1$) starting at the second piece of ultrasound data ($BM_2$), which is lastly stored during the sweep period, from the storage unit 150, as illustrated in FIG. 8.

The CM mode image forming unit 330 operates to form the CM mode image based on the first pieces of ultrasound data extracted by the extraction unit 320. In one embodiment, when the first pieces of ultrasound data ($CM_{18}$-$CM_7$) extracted by the extraction unit 320 are provided, the CM mode image forming unit 330 operates to calculate mean power, mean velocity and variance corresponding to the CM mode image based on the first pieces of ultrasound data ($CM_{18}$-$CM_7$) and forms the CM mode image using the calculated mean power (P), mean velocity (v) and variance ($\sigma^2$). By way of a non-limiting example, mean power, mean velocity and variance are calculated according to the following equation (1).

$$P=\hat{R}[0]=\frac{1}{N}\sum_{n=0}^{n=N-1} x[n]x[n]^*$$

$$v=v_{\max}\frac{\tan^{-1}(Im(\hat{R}[1])/Re(\hat{R}[1]))}{\pi}$$

$$\hat{R}[1]=\frac{1}{N-1}\sum_{n=0}^{N-2} x[n]^*x[n+1]$$

$$\sigma^2=1-\frac{|\hat{R}[1]|}{\hat{R}[0]} \qquad (1)$$

In equation 1, R[0] denotes $0^{th}$ order autocorrelation, R[1] denotes $1^{th}$ order autocorrelation, "N" denotes the average number equal to or greater than 2, x[n] denotes the input signal, Im() denotes an imaginary part of a complex number, and Re() denotes a real part of the complex number.

In another embodiment, when the first pieces of ultrasound data ($CM_{16}$-$CM_7$) extracted by the extraction unit 320 are provided, the CM mode image forming unit 330 operates to calculate mean power, mean velocity and variance corresponding to the CM mode image using the first pieces of ultrasound data ($CM_{16}$-$CM_7$), and forms the CM mode image using the calculated mean power, mean velocity and variance. By way of a non-limiting example, mean power (P), mean velocity (v) and variance ($\sigma^2$) are calculated according to the following equation (2).

$$P=R[0]=\frac{1}{N-k}\sum_{n=0}^{M-1}x[n]x[n]^{*}+\sum_{n=M+K}^{N-1}x[n]x[n]^{*}$$

$$v=v_{max}\frac{\tan^{-1}(Im(R[1])/Re(R[1]))}{\pi}$$

$$R[1]=\frac{1}{N-K-1}\left(\sum_{n=0}^{M-2}x[n]^{*}x[n+1]+\sum_{n=M+K}^{N-2}x[n]^{*}x[n+1]\right)$$

$$\sigma^2=1-\frac{|R[1]|}{R[0]} \qquad (2)$$

In yet another embodiment, when the first pieces of ultrasound data ($CM_{16}$-$CM_7$) extracted by the extraction unit 320 are provided, the CM mode image forming unit 330 operates to obtain third pieces of ultrasound data ($CM_8$' and $CM_{16}$'), which were not provided at the sweep speed, with the use of interpolation of the first pieces of ultrasound data ($CM_{16}$-$CM_7$) as illustrated in FIG. 9. The CM mode image forming unit 330 further operates to calculate an error comparing the first pieces of ultrasound data ($CM_{16}$-$CM_7$) extracted by the extraction unit 320 with the third pieces of ultrasound data ($CM_8$' and $CM_{16}$') obtained by using the interpolation. If the calculated error is less than a predetermined threshold, then the CM mode image forming unit 330 operates to calculate mean power, mean velocity and variance using the first pieces of ultrasound data ($CM_{16}$-$CM_7$) extracted by the extraction unit 320 and the third pieces of ultrasound data ($CM_8$' and $CM_{16}$') obtained by using the interpolation, and forms the CM mode image using the calculated mean power, mean velocity and variance. Assuming that the number of third pieces of ultrasound data obtained by using the interpolation is K, the third pieces of ultrasound data obtained by using the interpolation are located at M, M+1, and the total number of the first and third pieces of ultrasound data is N. Under the above conditions, mean power ($P_{total}$), mean velocity ($v_{total}$) and variance ($\sigma^2_{total}$) may be calculated according to the following equation (3).

$$P_{total}=R[0]_{total}=\frac{1}{N-k}\sum_{n=0}^{M-1}x[n]x[n]^{*}+\sum_{n=M+K}^{N-1}x[n]x[n]^{*}+\frac{1}{k}\sum_{n=M}^{M+1}x[n]x[n]^{*}$$

$$v_{total}=v_{\max}\frac{\tan^{-1}(Im(R[1]_{total}/ReR[1]_{total})}{\pi}$$

$$R[1]_{total}=\frac{1}{N-K-1}\left(\sum_{n=0}^{M-2}x[n]^{*}x[n+1]+\sum_{n=M+K}^{N-2}x[n]^{*}x[n+1]\right)+\frac{1}{k}\sum_{n=M-1}^{M+K}x[n]^{*}x[n+1]$$

$$\sigma_{total}^{2}=1-\frac{|R[1]_{total}|}{R[0]_{total}}$$

$$P_{IP}=R[0]_{IP}=\frac{1}{k}\sum_{n=M}^{M+1}x[n]x[n]^{*}$$

$$v_{IP}=v_{\max}\frac{\tan^{-1}(Im(R[1]_{IP}/ReR[1]_{IP})}{\pi}$$

$$R[1]_{IP}=\frac{1}{k}\sum_{n=M-1}^{M+K}x[n]^{*}x[n+1]$$

$$\sigma_{IP}^{2}=1-\frac{|R[1]_{IP}|}{R[0]_{IP}} \qquad (3)$$

In equation (3), $P_{IP}$, $v_{IP}$ and $\sigma^2_{IP}$ respectively represent mean power, mean velocity and variance, which are calculated from the third pieces of ultrasound data ($CM_8$' and $CM_{16}$') obtained by using the interpolation.

If the calculated error is more than the predetermined threshold, then the CM mode image forming unit 330 operates to calculate mean power, mean velocity and variance only using the first pieces of ultrasound data ($CM_{16}$-$CM_7$) extracted by the extraction unit 320, and forms the CM mode image using the calculated mean power, mean velocity and variance.

The BM mode image forming unit 340 operates to form a BM mode image based on the second pieces of ultrasound data extracted by the extraction unit 320. In one extraction unit 320 is provided, the BM mode image forming unit 340 operates to form the BM mode image based on the second piece of ultrasound data ($BM_{18}$). In another embodiment, when the second pieces of ultrasound data ($BM_{18}$-$BM_7$) extracted by the extraction unit 320 are provided, the BM mode image forming unit 340 operates to form the BM mode image through performing, for example, line average to the second pieces of ultrasound data ($BM_{18}$-$BM_7$). In yet another embodiment, when the second pieces of ultrasound data ($BM_1$ and $BM_2$) extracted by the extraction unit 320 are provided, the BM mode image forming unit 340 operates to form the BM mode image through performing, for example, line average to the second pieces of ultrasound data ($BM_1$ and $BM_2$).

Referring back to FIG. 1, the display unit 170 operates to display the CM mode image and the BM mode image formed at the image processor 160 according to the control unit 130. The display unit further operates to display the B mode image 410, the color doppler mode image 420 and the M line 440, as illustrated in FIG. 4. In one embodiment, the display unit 170 includes a liquid crystal display (LCD), a cathode ray tube (CRT) or any other device capable of displaying an image.

## Claims

**1.** An ultrasound system (100), comprising:

an ultrasound data acquisition unit (140) configured to perform a first operation for providing first ultrasound data for forming a color M mode image and a second operation for providing second ultrasound data for forming a brightness M mode image;
a control unit (130) configured to control the ultrasound data acquisition unit (140) to perform the first operation by a number of times equal to an ensemble number and the second operation one or more times according to an acquisition pattern within a sweep period to respectively accumulate the first ultrasound data and the second ultrasound data provided from the ultrasound data acquisition unit; and
an image processor (160) configured to form the color M mode image based on the accumulated first ultrasound data and to form the brightness M mode image based on the accumulated second ultrasound data,
**characterized in that**
the control unit (130) is further configured to calculate a time period expected for the ultrasound data acquisition unit (140) to perform the first and second operations once and to determine the acquisition pattern defining an order of performing the first and second operations based on the calculated time period, the sweep period, and the ensemble number, wherein the acquisition pattern is a pattern in which the first operation and the second operation are alternate, if the calculated time period is less than the sweep period divided by the ensemble number.

**2.** The ultrasound system (100) of Claim 1, wherein the ensemble number is programmable.

**3.** The ultrasound system (100) of Claim 1, wherein the image processor (160) is configured to form the brightness M mode image based on the second ultrasound data lastly provided by the ultrasound data acquisition unit (140) during the sweep period.

**4.** The ultrasound system (100) of Claim 1, wherein the image processor (160) is configured to form the brightness M mode image based on the second ultrasound data provided by the ultrasound data acquisition unit (140) during the sweep period.

**5.** The ultrasound system (100) of Claim 1, wherein the acquisition pattern is a pattern in which the first operation is performed by the number of times and then the second operation is performed once, if a time period expected for the ultrasound data acquisition unit (140) to perform the first and second operations once respectively is equal to or more than the sweep period divided by the ensemble number.

**6.** The ultrasound system (100) of Claim 5, wherein the image processor (160) is configured to form the color M mode image based on the first ultrasound data provided by the ultrasound data acquisition unit during the sweep period and to form the brightness M mode image based on the second ultrasound data provided by the ultrasound data acquisition unit (140) during the sweep period.

**7.** The ultrasound system (100) of Claim 5, wherein the image processor (160) is configured to obtain a third ultrasound data by using interpolation of the first ultrasound data provided by the ultrasound data acquisition unit (140) during the sweep period and form the color M mode image based on the first ultrasound data and the third ultrasound data.

**8.** A method of providing a color M mode image and a brightness M mode image, comprising:

a) performing at an ultrasound data acquisition unit (140) a first operation for providing first ultrasound data for forming a color M mode image and a second operation for providing second ultrasound data for forming a brightness M mode image;
b) controlling the ultrasound data acquisition unit (140) to perform the first operation a number of times equal to an ensemble number and the second operation one or more times according to an acquisition pattern within a sweep period to respectively accumulate the first ultrasound data and the second ultrasound data provided from the ultrasound data acquisition unit;

c) forming the color M mode image based on the first ultrasound data; and
d) forming the brightness M mode image based on the second ultrasound data, the method being **characterized by** the additional steps:
e) calculating a time period expected for the ultrasound data acquisition unit (140) to perform the first and second operations once and
f) determining the acquisition pattern defining an order of performing the first and second operations based on the calculated time period, the sweep period, and the ensemble number, wherein the acquisition pattern is a pattern in which the first operation and the second operation alternate, if the calculated time period is less than the sweep period divided by the ensemble number.

**9.** The method of Claim 8, wherein forming the brightness M mode image further comprises:

forming the brightness M mode image based on the second ultrasound data lastly provided by the ultrasound data acquisition unit during the sweep period.

**10.** The method of Claim 8, wherein forming the brightness M mode image further comprises:

forming the brightness M mode image based on the second ultrasound data provided by the ultrasound data acquisition unit during the sweep period.

**11.** The method of Claim 8, wherein the acquisition pattern is a pattern in which the first operation is performed by the number of times and then the second operation is performed once, if the calculated time period is equal to or more than the sweep period divided by the ensemble number.

**12.** The method of Claim 11, wherein forming the color M mode image further comprises:

forming the color M mode image based on the first ultrasound data provided by the ultrasound data acquisition unit during the sweep period; and
wherein forming the brightness M mode image further comprises:

forming the brightness M mode image based on the second ultrasound data provided by the ultrasound data acquisition unit during the sweep period.

**13.** The method of Claim 11, wherein forming the color M mode image further comprises:

obtaining a third ultrasound data by using interpolation of the first ultrasound data provided by the ultrasound data acquisition unit during the sweep period; and
forming the color M mode image based on the first ultrasound data and the third ultrasound data.

**Patentansprüche**

**1.** Ultraschallsystem (100), welches folgendes aufweist:

eine Ultraschalldaten-Beschaffungseinheit (140), die dafür vorgesehen ist, einen ersten Vorgang zur Bereitstellung erster Ultraschalldaten zum Bilden bzw. Erzeugen eines Farb-M-Modus-Bilds und einen zweiten Vorgang zur Bereitstellung zweiter Ultraschalldaten zum Bilden bzw. Erzeugen eines Helligkeits-M-Modus-Bilds durchzuführen;
eine Steuereinheit (130), die dafür vorgesehen ist, die Ultraschalldaten-Beschaffungseinheit (140) zu steuern, um den ersten Vorgang mehrere Male gleich einer Ensemblenummer bzw. einer ganzen Zahl und den zweiten Vorgang ein- oder mehrmals gemäß eines Beschaffungsmusters innerhalb einer Suchperiode durchzuführen, um jeweils die ersten Ultraschalldaten und die zweiten Ultraschalldaten, die von der Ultraschalldaten-Beschaffungseinheit bereitgestellt wurden, zu sammeln; und
einen Bildprozessor (160), der dafür vorgesehen ist, das Farb-M-Modus-Bild basierend auf den gesammelten ersten Ultraschalldaten zu bilden, und das Helligkeits-M-Modus-Bild basierend auf den gesammelten zweiten Ultraschalldaten zu bilden,
**dadurch gekennzeichnet, dass**
die Steuereinheit (130) des Weiteren dafür vorgesehen ist, eine Zeitperiode zu berechnen, von der erwartet wird, dass die Ultraschalldaten-Beschaffungseinheit (140) in derselben die ersten und zweiten Vorgänge einmal durchführt, und das Beschaffungsmuster zu ermitteln, welches eine Reihenfolge der Ausführung der ersten und zweiten Vorgänge basierend auf der berechneten Zeitperiode, der Suchperiode und der Ensemblenummer bzw. ganzen Zahl definiert, wobei das Beschaffungsmuster ein Muster ist, in welchem der erste Vorgang und der zweite Vorgang sich abwechseln, falls die berechnete Zeitperiode geringer ist als die Suchperiode geteilt durch die Ensemblenummer bzw. ganzen Zahl.

**2.** Ultraschallsystem (100) nach Anspruch 1, wobei die Ensemblenummer bzw. ganze Zahl programmierbar ist.

**3.** Ultraschallsystem (100) nach Anspruch 1, wobei der Bildprozessor (160) dafür vorgesehen ist, das Helligkeits-M-Modus-Bild basierend auf den zweiten Ultraschalldaten zu bilden, die zuletzt durch die Ultraschalldaten-Beschaffungseinheit (140) während der Suchperiode bereitgestellt wurden.

**4.** Ultraschallsystem (100) nach Anspruch 1, wobei der Bildprozessor (160) dafür vorgesehen ist, das Helligkeits-M-Modus-Bild basierend auf den zweiten Ultraschalldaten zu bilden, die durch die Ultraschalldaten-Beschaffungseinheit (140) während der Suchperiode bereitgestellt wurden.

**5.** Ultraschallsystem (100) nach Anspruch 1, wobei das Beschaffungsmuster ein Muster ist, in dem der erste Vorgang mehrere Male durchgeführt wird und der zweite Vorgang einmal durchgeführt wird, falls eine Zeitperiode, von der erwartet wird, dass die Ultraschalldaten-Beschaffungseinheit (140) in derselben die ersten und zweiten Vorgänge jeweils einmal durchführt, gleich oder mehr als die Suchperiode geteilt durch die Ensemblenummer bzw. ganze Zahl ist.

**6.** Ultraschallsystem (100) nach Anspruch 5, wobei der Bildprozessor (160) dafür vorgesehen ist, das Farb-M-Modus-Bild basierend auf den ersten Ultraschalldaten zu bilden, die von der Ultraschalldaten-Beschaffungseinheit (140) während der Suchperiode bereitgestellt wurden, und das Helligkeits-M-Modus-Bild basierend auf den zweiten Ultraschalldaten zu bilden, welche durch die Ultraschalldaten-Beschaffungseinheit (140) während der Suchperiode bereitgestellt wurden.

**7.** Ultraschallsystem (100) nach Anspruch 5, wobei der Bildprozessor (160) dafür vorgesehen ist, dritte Ultraschalldaten durch Verwenden einer Interpolation der ersten Ultraschalldaten, die von der Ultraschalldaten-Beschaffungseinheit (140) während der Suchperiode bereitgestellt wurden, zu erlangen, und das Farb-M-Modus-Bild basierend auf den ersten Ultraschalldaten und den dritten Ultraschalldaten zu bilden.

**8.** Verfahren zur Bereitstellung eines Farb-M-Modus-Bilds und eines Helligkeits-M-Modus-Bilds, welches Folgendes aufweist:

a) Durchführen eines ersten Vorgangs zur Bereitstellung erster Ultraschalldaten zum Bilden bzw. Erzeugen eines Farb-M-Modus-Bilds und eines zweiten Vorgangs zur Bereitstellung zweiter Ultraschalldaten zum Bilden bzw. Erzeugen eines Helligkeits-M-Modus-Bilds in einer Ultraschalldaten-Beschaffungseinheit (140);
b) Steuern der Ultraschalldaten-Beschaffungseinheit (140), um den ersten Vorgang mehrere Male entsprechend einer Ensemblenummer bzw. einer ganzen Zahl und den zweiten Vorgang ein- oder mehrmals gemäß eines Beschaffungsmusters innerhalb einer Suchperiode durchzuführen, um die ersten Ultraschalldaten und die zweiten Ultraschalldaten, die von der Ultraschalldaten-Beschaffungseinheit bereitgestellt wurden, zu sammeln;
c) Bilden des Farb-M-Modus-Bilds basierend auf den ersten Ultraschalldaten; und
d) Bilden des Helligkeits-M-Modus-Bilds basierend auf den zweiten Ultraschalldaten,
wobei das Verfahren durch die folgenden zusätzlichen Schritte gekennzeichnet ist:
e) Berechnen einer Zeitperiode, von der erwartet wird, dass die Ultraschalldaten-Beschaffungseinheit (140) in derselben die ersten und zweiten Vorgänge einmal durchführt, und
f) Ermitteln des Beschaffungsmusters, welches eine Reihenfolge der Durchführung der ersten und zweiten Vorgänge basierend auf der berechneten Zeitperiode, der Suchperiode und der Ensemblenummer bzw. ganzen Zahl definiert, wobei das Beschaffungsmuster ein Muster ist, in welchem der erste Vorgang und der zweite Vorgang sich abwechseln, falls die berechnete Zeitperiode geringer ist als die Suchperiode geteilt durch die Ensemblenummer bzw. ganze Zahl.

**9.** Verfahren nach Anspruch 8, wobei das Bilden des Helligkeits-M-Modus-Bilds des Weiteren Folgendes aufweist:

Bilden des Helligkeits-M-Modus-Bilds basierend auf den zweiten Ultraschalldaten, die zuletzt durch die Ultraschalldaten-Beschaffungseinheit während der Suchperiode bereitgestellt wurden.

**10.** Verfahren nach Anspruch 8, wobei das Bilden des Helligkeits-M-Modus-Bilds des Weiteren Folgendes aufweist:

Bilden des Helligkeits-M-Modus-Bilds basierend auf den zweiten Ultraschalldaten, die durch die Ultraschalldaten-Beschaffungseinheit während der Suchperiode bereitgestellt wurden.

**11.** Verfahren nach Anspruch 8, wobei das Beschaffungsmuster ein Muster ist, in dem der erste Vorgang mehrere Male durchgeführt wird und der zweite Vorgang einmal durchgeführt wird, falls die berechnete Zeitperiode gleich oder mehr als die Suchperiode geteilt durch die Ensemblenummer bzw. ganze Zahl ist.

**12.** Verfahren nach Anspruch 11, wobei das Bilden des Farb-M-Modus-Bilds des Weiteren Folgendes aufweist:

Bilden des Farb-M-Modus-Bilds basierend auf den ersten Ultraschalldaten, die von der Ultraschalldaten-Beschaffungseinheit während der Suchperiode bereitgestellt wurden; und
wobei das Bilden des Helligkeits-M-Modus-Bilds des Weiteren Folgendes aufweist:

Bilden des Helligkeits-M-Modus-Bilds basierend auf den zweiten Ultraschalldaten, welche durch die Ultraschalldaten-Beschaffungseinheit während der Suchperiode bereitgestellt wurden.

**13.** Verfahren nach Anspruch 11, wobei das Bilden des Farb-M-Modus-Bilds des Weiteren Folgendes aufweist:

Erlangen von dritten Ultraschalldaten durch Verwenden einer Interpolation der ersten Ultraschalldaten, die von der Ultraschalldaten-Beschaffungseinheit während der Suchperiode bereitgestellt wurden; und
Bilden des Farb-M-Modus-Bilds basierend auf den ersten Ultraschalldaten und den dritten Ultraschalldaten.

## Revendications

**1.** Système ultrasonique (100), comportant :

une unité d'acquisition de données (140) configurée pour effectuer une première opération en vue de fournir des premières données ultrasoniques pour réaliser une image M en mode couleur et une seconde opération en vue de fournir des secondes données ultrasoniques pour réaliser une image M en mode de brillance ;
une unité de commande (130) configurée pour contrôler
l'unité d'acquisition des données ultrasoniques (140) pour réaliser la première opération un nombre de fois égal à tous les nombres et la seconde opération une ou plusieurs fois selon que acquisitions de trames dans périodes de balayage pour accumuler respectivement les premières données ultrasoniques et les secondes données ultrasoniques fournies par l'unité d'acquisition des données ultrasoniques ; et
un processeur d'images (160) configuré pour former l'image M en mode couleur basée sur les premières données ultrasoniques accumulées et pour former pour former l'image M en mode de brillance basée sur les secondes données ultrasoniques accumulées,

**caractérisé en ce que**
l'unité de commande (130) est en outre configurée pour calculer une période de temps prévu pour que l'unité d'acquisition de données (140) puisse effectuer d'une part la première et la seconde opérations et pour déterminer la trame d'acquisition définissant un ordre de réalisation des première et seconde opérations basé sur la période de temps calculée, la période de balayage, et le nombre total, dans lequel, la trame d'acquisition est une trame dans laquelle la première opération et la seconde opération sont alternées si la période calculée est inférieure à la période de balayage divisée par le nombre total.

**2.** Système ultrasonique (100) selon la revendication 1, dans lequel le nombre total est programmable.

**3.** Système ultrasonique (100) selon la revendication 1, dans lequel le processeur d'image (160) est configuré pour former une image M en mode brillance basée sur les secondes données ultrasoniques fournies en dernier par l'unité d'acquisition de données ultrasoniques (140) pendant la période de balayage.

**4.** Système ultrasonique (100) selon la revendication 1, dans lequel le processeur d'image (160) est configuré pour former une image M en mode brillance basée sur les secondes données ultrasoniques fournies par l'unité d'acquisition de données ultrasoniques (140) pendant la période de balayage.

**5.** Système ultrasonique (100) selon la revendication 1, dans lequel la trame d'acquisition est une trame dans laquelle la première opération est effectuée un certain nombre de fois et la seconde opération est effectuée une fois, si la période de temps envisagée pour que l'unité d'acquisition de données ultrasoniques (140) effectue la première et la seconde opération, respectivement une fois est égale ou supérieure à la période de balayage divisée par le nombre total.

**6.** Système ultrasonique (100) selon la revendication 5, dans lequel le processeur d'image (160) est configuré pour former une image M en mode couleur basée sur les premières données ultrasoniques fournies par l'unité d'acquisition de données ultrasoniques pendant la période de balayage et pour former une image M en mode brillance, basée sur les secondes données ultrasoniques fournies par l'unité d'acquisition de données ultrasoniques (140) pendant la période de balayage.

**7.** Système ultrasonique (100) selon la revendication 5, dans lequel le processeur d'image (160) est configuré pour obtenir des troisièmes données ultrasoniques en effectuant une interpolation des premières données ultrasoniques de l'unité d'acquisition de données ultrasoniques (140) pendant la période de balayage et en formant l'image M en mode couleur basées sur les premières données ultrasoniques et sur les troisièmes données ultrasoniques.

**8.** Procédé pour réaliser une image M en mode couleur et une image M en mode brillance, consistant à :

a) effectuer, sur une unité d'acquisition de données ultrasoniques (140), une première opération pour obtenir des premières données ultrasoniques pour former une image M en mode couleur et une seconde opération pour obtenir des secondes données ultrasoniques pour former une image M en mode brillance ;
b) commander l'unité d'acquisition de données ultrasoniques (140) pour effectuer la première opération un nombre de fois égal au nombre total et la seconde opération une ou plusieurs fois selon les trames d'acquisition dans la période de balayage pour accumuler respectivement les premières données ultrasoniques et les secondes données ultrasoniques de l'unité d'acquisition de données ultrasoniques;
c) réaliser l'image M en mode couleur basées sur les premières données ultrasoniques ; et
d) réaliser l'image M en mode brillance basées sur les secondes données ultrasoniques,
le procédé étant **caractérisé par** les étapes additionnelles consistant à :
e) calculer une période de temps prévu pour que l'unité d'acquisition de données (140) puisse effectuer une fois la première et la seconde opération et
f) déterminer la trame d'acquisition définissant un ordre de réalisation des première et seconde opérations basé sur la période de temps calculée, la période de balayage, et le nombre total, dans lequel, la trame d'acquisition est une trame dans laquelle la première opération et la seconde opération sont alternées si la période calculée est inférieure à la période de balayage divisée par le nombre total.

**9.** Procédé selon la revendication 8, dans lequel la formation de l'image M en mode brillance comporte en outre :

la formation de l'image M en mode brillance basée sur les secondes données ultrasoniques fournies en dernier par l'unité d'acquisition de données pendant la période de balayage.

**10.** Procédé selon la revendication 8, dans lequel la formation de l'image M en mode brillance comporte en outre :

la formation de l'image M en mode brillance basée sur les secondes données ultrasoniques fournies par l'unité d'acquisition de données pendant la période de balayage.

**11.** Procédé selon la revendication 8, dans lequel la trame d'acquisition est une trame dans laquelle la première opération est effectuée le nombre de fois et ensuite la seconde opération est effectuée une fois, si la période de temps calculée est égale ou supérieure à la période de balayage divisée par le nombre total.

**12.** Procédé selon la revendication 11, dans lequel la création de l'image M en mode couleur consiste en outre :

à former une image M en mode couleur basée sur les premières données ultrasoniques fournies par l'unité d'acquisition de données ultrasoniques pendant la période de balayage ; et
dans lequel l'obtention d'une image M en mode brillance, consiste en outre :

à former l'image M en mode brillance basée sur les secondes données ultrasoniques fournies par l'unité d'acquisition de données ultrasoniques pendant la période de balayage.

**13.** Procédé selon la revendication 11, dans lequel la création de l'image M en mode couleur consiste en outre :

à obtenir des troisièmes données ultrasoniques en effectuant une interpolation des premières données ultrasoniques de l'unité d'acquisition de données ultrasoniques pendant la période de balayage ; et
à former l'image M en mode couleur basées sur les premières données ultrasoniques et sur les troisièmes données ultrasoniques.

FIG. 1

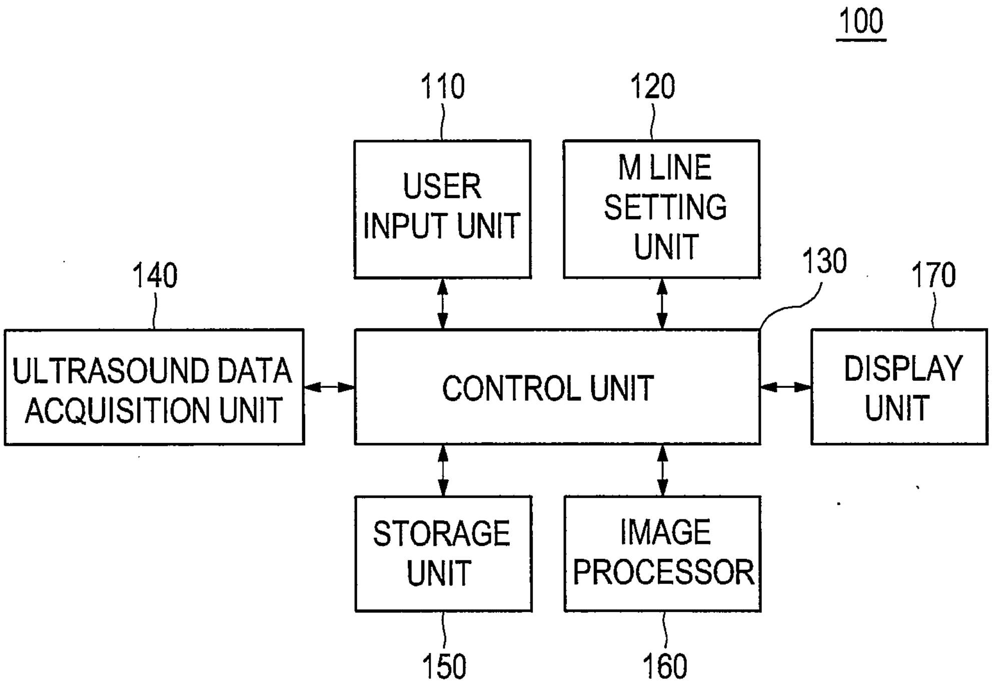
100
110
USER INPUT UNIT
120
M LINE SETTING UNIT
140
ULTRASOUND DATA ACQUISITION UNIT
130
CONTROL UNIT
170
DISPLAY UNIT
STORAGE UNIT
150
IMAGE PROCESSOR
160

FIG. 2

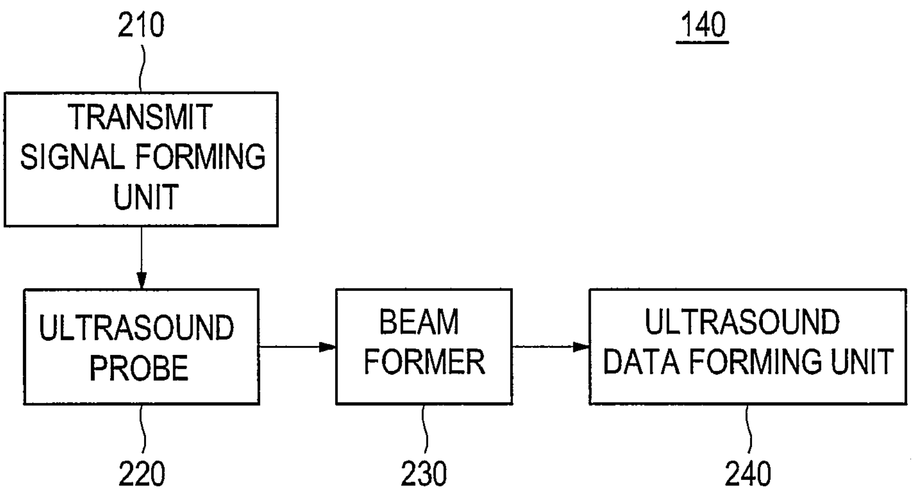
210
TRANSMIT SIGNAL FORMING UNIT
140
ULTRASOUND PROBE
220
BEAM FORMER
230
ULTRASOUND DATA FORMING UNIT
240

FIG. 3

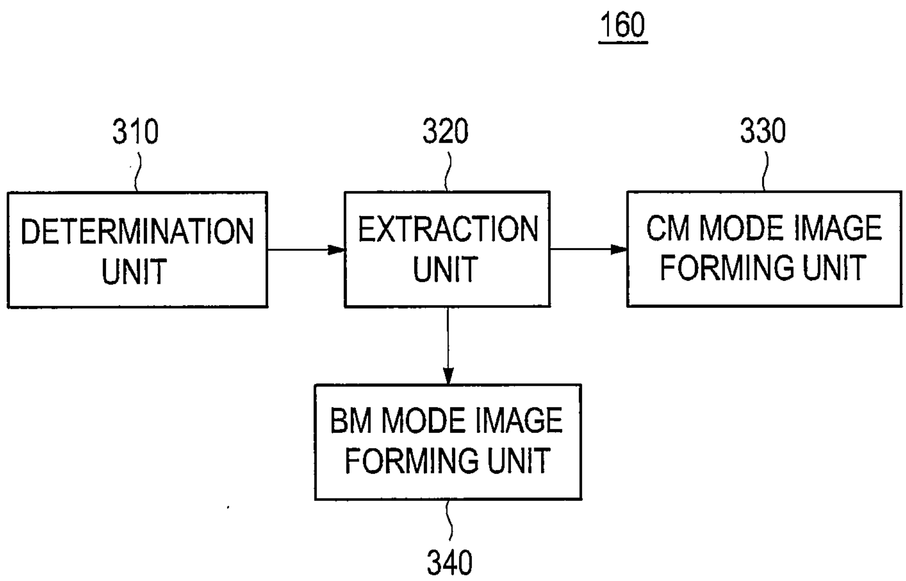
160
310
320
330
DETERMINATION UNIT
EXTRACTION UNIT
CM MODE IMAGE FORMING UNIT
BM MODE IMAGE FORMING UNIT
340

FIG. 4

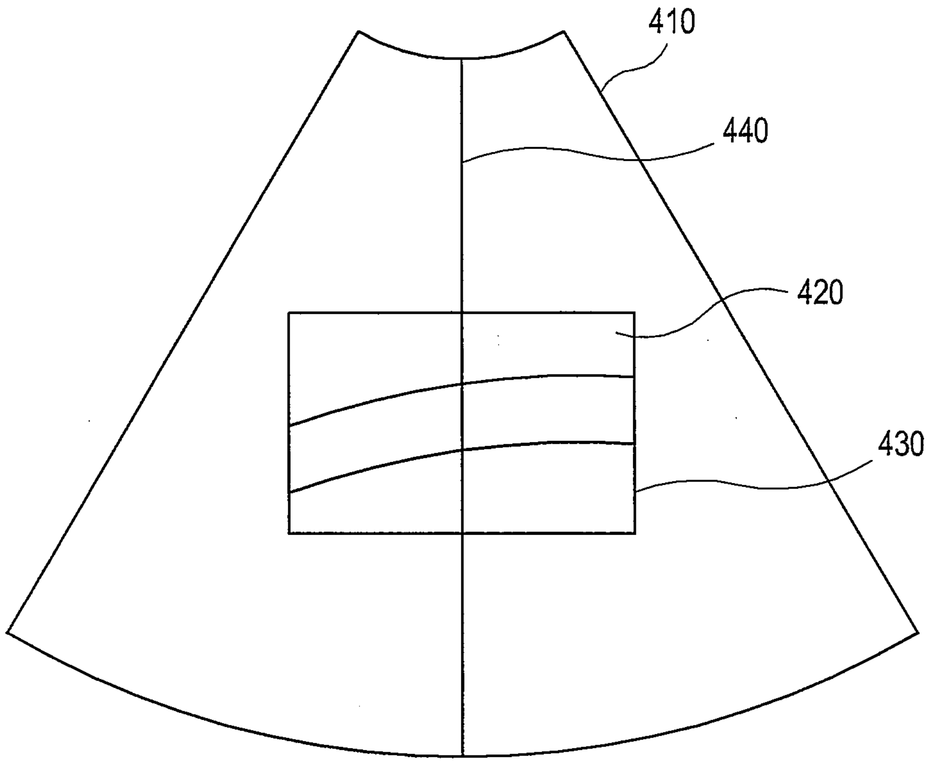

FIG. 5

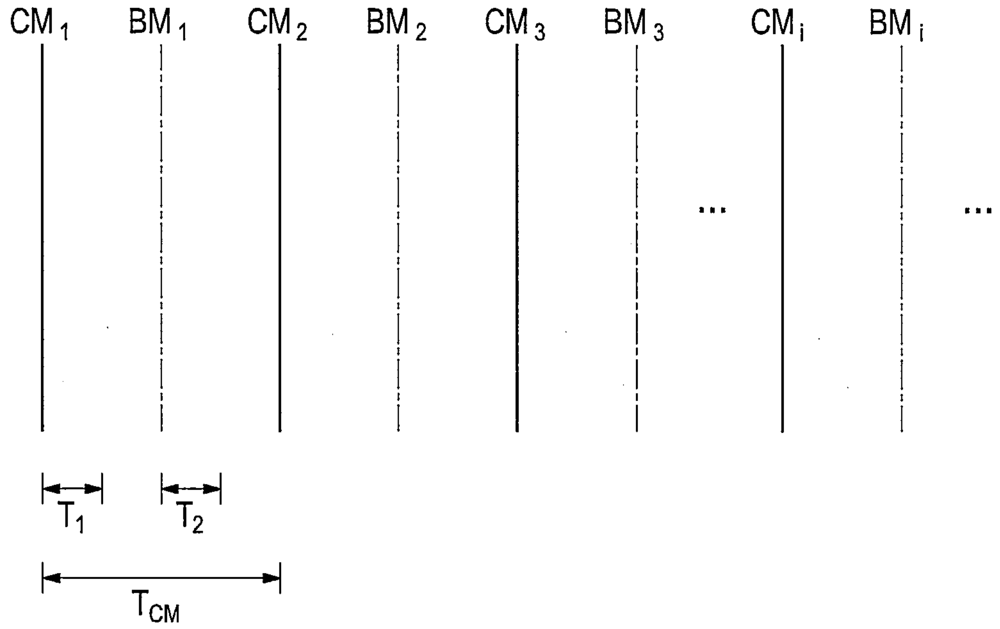
CM 1
BM 1
CM 2
BM 2
CM 3
BM 3
CM i
BM i
...
...
T1
T2
TCM

FIG. 6

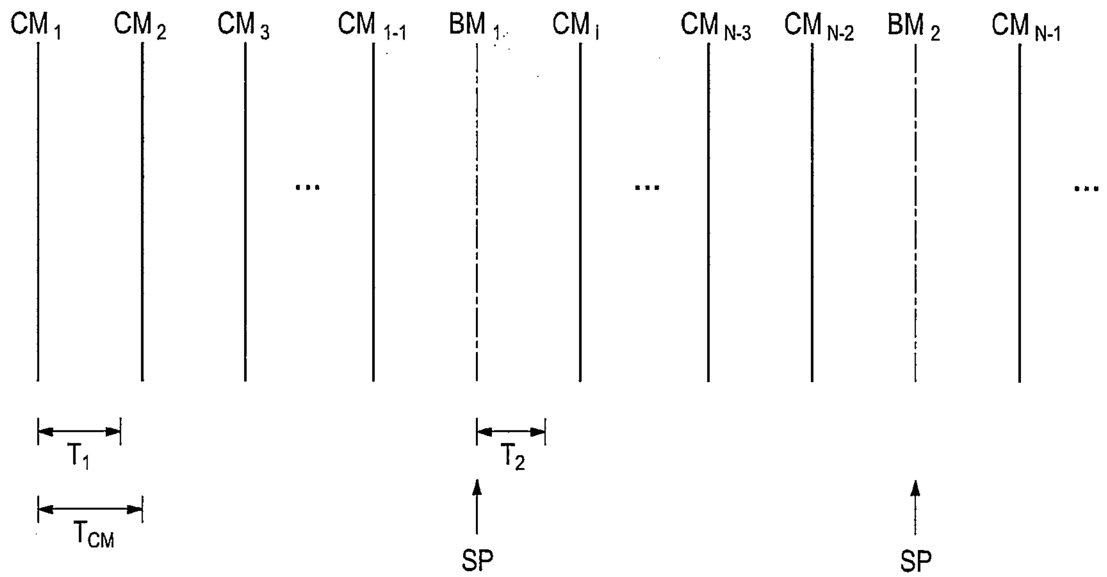
CM 1
CM 2
CM 3
CM 1-1
BM 1
CM i
CM N-3
CM N-2
BM 2
CM N-1
...
...
...
T1
TCM
T2
SP
SP

FIG. 7

| ORDER | FIRST ULTRASOUND DATA | SECOND ULTRASOUND DATA | |
|---|---|---|---|
| 1 | $CM_1$ | $BM_1$ | |
| 2 | $CM_2$ | $BM_2$ | |
| 3 | $CM_3$ | $BM_3$ | |
| 4 | $CM_4$ | $BM_4$ | |
| 5 | $CM_5$ | $BM_5$ | |
| 6 | $CM_6$ | $BM_6$ | |
| 7 | $CM_7$ | $BM_7$ | |
| 8 | $CM_8$ | $BM_8$ | |
| 9 | $CM_9$ | $BM_9$ | |
| 10 | $CM_{10}$ | $BM_{10}$ | |
| 11 | $CM_{11}$ | $BM_{11}$ | |
| 12 | $CM_{12}$ | $BM_{12}$ | |
| 13 | $CM_{13}$ | $BM_{13}$ | |
| 14 | $CM_{14}$ | $BM_{14}$ | |
| 15 | $CM_{15}$ | $BM_{15}$ | |
| 16 | $CM_{16}$ | $BM_{16}$ | |
| 17 | $CM_{17}$ | $BM_{17}$ | |
| 18 | $CM_{18}$ | $BM_{18}$ | ← SP |
| 19 | $CM_{19}$ | $BM_{19}$ | |
| ⋮ | ⋮ | ⋮ | |

FIG. 8

| ORDER | FIRST ULTRASOUND DATA | SECOND ULTRASOUND DATA | |
|---|---|---|---|
| 1 | $CM_1$ | | |
| 2 | $CM_2$ | | |
| 3 | $CM_3$ | | |
| 4 | $CM_4$ | | |
| 5 | $CM_5$ | | |
| 6 | $CM_6$ | | |
| 7 | $CM_7$ | | |
| 8 | $CM_8$ | | |
| 9 | | $BM_1$ | ← SP |
| 10 | $CM_9$ | | |
| 11 | $CM_{10}$ | | |
| 12 | $CM_{11}$ | | |
| 13 | $CM_{12}$ | | |
| 14 | $CM_{13}$ | | |
| 15 | $CM_{14}$ | | |
| 16 | $CM_{15}$ | | |
| 17 | $CM_{16}$ | | |
| 18 | | $BM_2$ | ← SP |
| 19 | $CM_{17}$ | | |
| ⋮ | ⋮ | ⋮ | |

FIG. 9

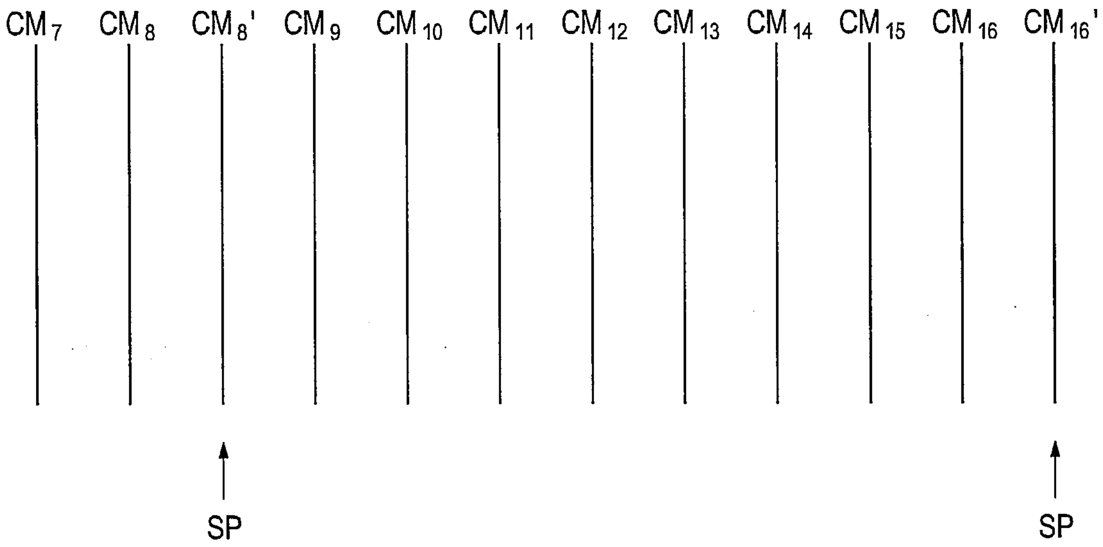

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1498746 A1
- US 20030045795 A1
- US 4817617 A